Europäisches Patentamt

(19) European Patent Office

Office européen des brevets

(11) EP 0 734 246 B1

(12) EUROPÄISCHE PATENTSCHRIFT

(45) Veröffentlichungstag und Bekanntmachung des Hinweises auf die Patenterteilung:
27.01.1999 Patentblatt 1999/04

(21) Anmeldenummer: 95900643.8

(22) Anmeldetag: 18.11.1994

(51) Int. Cl.$^6$: **A61K 7/42**, A61K 7/48

(86) Internationale Anmeldenummer:
PCT/DE94/01363

(87) Internationale Veröffentlichungsnummer:
WO 95/17160 (29.06.1995 Gazette 1995/27)

(54) **KOSMETISCHE ODER DERMATOLOGISCHE O/W-EMULSIONEN, ENTHALTEND AMINOSÄUREN UND ANORGANISCHE PIGMENTE**

COSMETIC OR DERMATOLOGICAL OIL-IN-WATER EMULSIONS CONTAINING AMINO ACIDS AND INORGANIC PIGMENTS

EMULSIONS COSMETIQUES OU DERMATOLOGIQUES HUILE-DANS-L'EAU CONTENANT DES ACIDES AMINES ET DES PIGMENTS INORGANIQUES

(84) Benannte Vertragsstaaten:
**AT BE CH DE ES FR GB IT LI NL**

(30) Priorität: 15.12.1993 DE 4342719

(43) Veröffentlichungstag der Anmeldung:
02.10.1996 Patentblatt 1996/40

(73) Patentinhaber:
**Beiersdorf Aktiengesellschaft**
**20245 Hamburg (DE)**

(72) Erfinder:
• **GERS-BARLAG, Heinrich**
**D-25459 Kummerfeld (DE)**
• **NISSEN, Bente**
**D-20145 Hamburg (DE)**
• **RUDOLPH, Martin**
**D-22397 Hamburg (DE)**

(56) Entgegenhaltungen:
**EP-A- 0 193 387**     **EP-A- 0 313 304**
**EP-A- 0 456 458**     **DE-U- 9 104 777**
**US-A- 5 254 331**

• **DATABASE WPI Week 8316, Derwent Publications Ltd., London, GB; AN 78-88546A & JP,A,53 124 627 (SHISEIDO)**
• **DATABASE WPI Week 9345, Derwent Publications Ltd., London, GB; AN 93-358162 & SU,A,1 770 352 (AEROZOL SCI PRODN ASSOC)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist. (Art. 99(1) Europäisches Patentübereinkommen).

**Beschreibung**

Die vorliegende Erfindung betrifft kosmetische bzw. dermatologische O/W-Emulsionen mit einem Gehalt an Aminosäuren und anorganischen Pigmenten sowie die Verwendung solcher O/W-Emulsionen auf dem Gebiete der kosmetischen und dermatologischen Hautpflege.

Insbesondere betrifft die Erfindung Zubereitungen mit extrem niedrigem sogenanntem "Stinging Potential".

Unter Hautpflege ist in erster Linie zu verstehen, daß die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliche Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Zeichen der Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen, also metastabile Zwei- oder Mehrphasensysteme bei welchen die einzelnen Phasen im flüssigen Zustande vorliegen. Ein gängiger Emulsionstyp sind O/W-Emulsionen.

Die Epidermis ist reich mit Nerven und Nervenendapparaten wie Vater-Pacini-Lamellenkörpern, Merkel-Zell-Neuritenkomplexen und freien Nervenendigungen für Schmerz-, Kälte-, Wärmeempfindung und Juckreiz ausgestattet.

Bei Menschen mit sensibler, empfindlicher oder verletzlicher Haut kann ein mit "Stinging" (〈engl.〉 "to sting" = verletzen, brennen, schmerzen) bezeichnetes neurosensorisches Phänomen beobachtet werden. Diese "sensible Haut" unterscheidet sich grundsätzlich von "trockener Haut" mit verdickten und verhärteten Hornschichten.

Typische Reaktionen des "Stinging" bei sensibler Haut sind Rötung, Spannen und Brennen der Haut sowie Juckreiz.

Als neurosensorisches Phänomen ist der Juckreiz bei atopischer Haut anzusehen, sowie Juckreiz bei Hauterkrankungen.

"Stinging"-Phänomene können als kosmetisch zu behandelnde Störungen angesehen werden. Starker Juckreiz dagegen, insbesondere bei Atopie auftretendes starkes Hautjucken, kann auch als schwerwiegendere dermatologische Störung bezeichnet werden.

Typische, mit den Begriffen "Stinging" oder "empfindlicher Haut" in Verbindung gebrachte, störende neurosensorische Phänomene sind Hautrötung, Kribbeln, Prickeln, Spannen und Brennen der Haut und Juckreiz. Sie können durch stimulierende Umgebungsbedingungen z.B. Massage, Tensideinwirkung, Wettereinfluß wie Sonne, Kälte, Trockenheit, aber auch feuchte Wärme, Wärmestrahlung und UV-Strahlung, z.B. der Sonne, hervorgerufen werden.

In "Journal of the Society of Cosmetic Chemists" 28, S.197 - 209 (Mai 1977) beschreiben P.J.Frosch und A.M.Kligman eine Methode zur Abschätzung des "Stinging-Potentials" topisch verabreichter Substanzen. Als positive Substanzen werden hier z.B. Milchsäure und Brenztraubensäure eingesetzt. Bei Messung nach dieser Methode wurden aber auch Aminosäuren, insbesondere Glycin, als neurosensorisch aktiv ermittelt (solche Substanzen werden "Stinger" genannt).

Nach bisherigen Erkenntnissen tritt eine derartige Empfindlichkelt gegenüber ganz bestimmten Substanzen individuell unterschiedlich auf. Dies bedeutet, eine Person, die bei Kontakt mit einer Aminosäure "Stingingeffekte" erlebt, wird sie mit hoher Wahrscheinlichkeit bei jedem weiteren Kontakt wiederholt erleben. Der Kontakt mit anderen "Stingern" kann aber ohne jede Reaktion verlaufen.

Aminosäuren sind Bestandteil des natürlichen Feuchtigkeitsfaktors (der sogenannte Natural Moisturizing Factor). Der Zusatz von Aminosäuren, insbesondere essentieller Aminosäuren, ist als vorteilhaft anzusehen, da über Hydratationsvorgänge Feuchtigkeit in der Haut gebunden werden kann.

Aminosäuren mit besonders vorteilhafter kosmetischer bzw. dermatologischer Wirkung sind Glycin, Alanin, Valin, Leucin, Isoleucin, Phenylalanin, Tyrosin, Prolin, Hydroxyprolin, Serin, Threonin, Cystein, Methionin, Tryptophan, Arginin.

Die Einsatzmenge solcher Aminosäuren, insbesondere die von Glycin, ist jedoch begrenzt, da bei empfindlichen Personen bereits bei Konzentrationen unterhalb von 0,5 Gew.-% das vorab beschriebene "Stinging" auftreten kann.

Da es, wie vorab beschrieben, aber wünschenswert ist, auch empfindlichen Personen die kosmetische oder dermatologische Verabreichung von Aminosäuren zu ermöglichen, war es eine Aufgabe der vorliegenden Erfindung, kosmetische oder dermatologische Zubereitungen zu entwickeln, welche zwar Aminosäuren enthalten, sich aber durch ein extrem niedriges "Stinging-Potential" auszeichnen, günstigenfalls praktisch frei von "Stingingeffekten" sein sollten.

Es hat sich erstaunlicherweise vorausgestellt, und darin liegt die Lösung der Aufgabe begründet, daß

O/W-Emulsionen, enthaltend Gemische aus hydrophoben anorganischen Pigmenten und Aminosäuren,

bzw. die Verwendung von Gemischen aus hydrophoben anorganischen Pigmenten und Aminosäuren in O/W-Emulsionen zur Verhinderung des "Stingings",

bzw. die Verwendung hydrophober anorganischer Pigmente zur Verhinderung des "Stingings" von Aminosäuren in O/W-Emulsionen,

den Mißständen des Standes der Technik abhelfen.

Es war nicht vorherzusehen gewesen, daß bei Verwendung der erfindungsgemäßen Gemische nicht nur das "Stinging-Potential" der Aminosäuren selbst für empfindliche Personen praktisch auf Null reduziert werden, sondern daß darüberhinaus die volle Aktivität der Aminosäuren erhalten bleiben würde.

Es wird vermutet, daß die erfindungsgemäßen Aminosäuren an die Oberfläche der hydrophoben anorganischen Pigmente adsorbiert werden.

Zwar beschreiben M.Schmidt und S.G.Steinemann in "XPS studies of amino acids adsorbed on titanium dioxide surfaces", Fresenius' Journal of Analytical Chemistry (1991) 341, S.412 - 415, daß gewisse Aminosäuren leicht von der Oberfläche von Titandioxidpartikeln adsorbiert werden. Ein Hinweis auf die vorliegende Erfindung und ihre vorteilhaften Eigenschaften liefert diese Arbeit jedoch keineswegs. Zudem ist das $TiO_2$, welches a.a.O. zitiert wird, nicht hydrophob.

Im Gegenteil hätte die vorabzitierte Arbeit vermuten lassen, daß durch eine Adsorption der Aminosäuren die erwünschte Aktivität der Aminosäuren vermindert wird.

In unerwarteter Weise hat sich jedoch herausgestellt, daß die Aktivität (die sich beispielsweise in den ausgezeichneten Werten für die Hautfeuchtigkeit nach Anwendung Aminosäure enthaltender Formulierungen äußert) kein Unterschied zwischen Formulierungen ergibt, welche erfindungsgemäße Pigmente enthalten und solchen, welche frei davon sind.

Erfindungsgemäß werden Aminosäuren mit besonders vorteilhafter kosmetischer bzw. dermatologischer Wirkung gewählt aus der Gruppe Glycin, Alanin, Valin, Leucin, Isoleucin, Phenylalanin, Tyrosin, Prolin, Hydroxyprolin, Serin, Threonin, Cystein, Methionin, Tryptophan, Arginin.

Besonders bevorzugt von diesen Verbindungen sind Arginin und, ganz besonders bevorzugt, Glycin.

Erfindungsgemäße kosmetische und dermatologische Zubereitungen enthalten bevorzugt anorganische Pigmente auf Basis von Metalloxiden und/oder anderen in Wasser schwerlöslichen oder unlöslichen Metallverbindungen, insbesondere der Oxide des Titans ($TiO_2$), Zinks (ZnO), Eisens (z.B. $Fe_2O_3$), Zirkoniums ($ZrO_2$), Siliciums ($SiO_2$), Mangans (z.B. MnO), Aluminiums ($Al_2O_3$), Cers (z.B. $Ce_2O_3$), Mischoxiden der entsprechenden Metalle sowie Abmischungen aus solchen Oxiden. Besonders bevorzugt handelt es sich um Pigmente auf der Basis von $TiO_2$.

Voraussetzung für die Verwendbarkeit anorganischer Pigmente für die erfindungsgemäßen Zwecke ist natürlich die kosmetische bzw. dermatologische Unbedenklichkeit der zugrundeliegenden Substanzen.

Im wesentlichen unerheblich für die vorliegende Erfindung ist dabei, in welchen Modifikationen solche Metalloxide vorliegen. $TiO_2$ beispielsweise kommt in der Natur in drei Hauptmodifikationen (Rutil, Anatas und Brookit) vor, welche grundsätzlich alle gleichermaßen geeignet sind. Ähnliches gilt für die Modifikationen der Eisenoxide usw.

Vorteilhaft ist, den Partikeldurchmesser der verwendeten Pigmente kleiner als 100 nm zu wählen.

Erfindungsgemäß liegen die anorganischen Pigmente in hydrophober Form vor, d.h., daß sie oberflächlich wasserabweisend behandelt sind. Diese Oberflächenbehandlung kann darin bestehen, daß die Pigmente nach an sich bekannten Verfahren mit einer dünnen hydrophoben Schicht versehen werden.

Eines solcher Verfahren besteht beispielsweise darin, daß die hydrophobe Oberflächenschicht nach einer Reaktion gemäß

$$n\ TiO_2 + m\ (RO)_3Si\text{-}R' \rightarrow n\ TiO_2\ (oberfl.)$$

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte $TiO_2$-Pigmente sind beispielsweise unter den Handelsbezeichnungen T 805 von der Firma DEGUSSA oder M 262 von der Firma KEMIRA oder MT 100 T von der Firma TAYCA erhältlich.

Erfindungsgemäße O/W-Emulsionen sind vorteilhaft durch einen Gehalt von 0,1 bis 10 Gew.-%, insbesondere 0,5 - 3,0 Gew.-%, an Aminosäuren und/oder einen Gehalt von 0,1 bis 10 Gew.-%, insbesondere 0,5 - 5,0 Gew.-% an hydrophoben anorganischen Pigmenten gekennzeichnet, jeweils bezogen auf das Gesamtgewicht der Zusammensetzung.

Die Herstellung erfindungsgemäßer O/W-Emulsionen geschieht nach den üblichen, dem Fachmanne geläufigen Regeln.

Es ist möglich und vorteilhaft, das oder die hydrophoben anorganischen Pigmente und die Aminosäure oder die -säuren zu jedem beliebigen Zeitpunkte der Emulsionsherstellung dem Emulsionsgemisch zuzugeben. Dabei können Pigment oder Pigmente und Aminosäure oder -säuren sowohl getrennt als auch bereits miteinander vereinigt dem Emulsionsgemisch zugegeben werden.

Besonders vorteilhafte Zubereitungen werden ferner erhalten, wenn die erfindungsgemäßen Wirkstoffe mit Antioxidantien kombiniert werden. Erfindungsgemäß enthalten die O/W-Emulsionen vorteilhaft eines oder mehrere Antioxidantien. Als günstige, aber dennoch fakultativ zu verwendende Antioxidantien alle für kosmetische und/oder dermatologische Anwendungen geeigneten oder gebräuchlichen Antioxidantien verwendet werden.

Besonders vorteilhaft werden die Antioxidantien gewählt aus der Gruppe bestehend aus

Ascorbinsäure (Vitamin C), Ascorbinsäurederivaten, den verschiedenen Tocopherolen (Vitamin E) und Tocopherylestern bzw. anderen Tocopherolderivaten, Folsäure (früher als Vitamin $B_c$, $B_9$, oder M bezeichnet, heute der Vitamin $B_2$-Gruppe zugeordnet), Phytinsäure (Inosithexaphosphorsäure, auch Fytinsäure), den verschiedenen Ubichinonen (Mitochinone, Coenzyme Q), Gallenextrakt, cis- und/oder trans-Urocaninsäure (4-Imidazolylacrylsäure), Carnosin (N-β-Alanyl-L-histidin, Ignotin), Histidin, Flavonen oder Flavonoiden, Cystin (3,3'-Dithiobis(2-aminopropionsäure), Cystein (2-Amino-3-mercaptopropionsäure) und dessen Derivaten (z.B. N-Acetylcystein), die verschiedenen Carotine (insbesondere β-Carotin und Lycopin (Psi-Carotin)), Tyrosin (2-Amino-3-(4-Hydroxyphenyl)-propionsäure), α-Liponsäure (1,2-Dithiolan-3-pentansäure), Glutathion (gamma-L-Glutamyl-L-cysteinglycin) und Glutathionester, Furalglucitol (Sorbitylfurfural), Mannit sowie Zinkoxid und Zinksalze (z.B.$ZnSO_4$).

Es war nicht vorauszusehen gewesen, daß die erfindungsgemäße Kombination aus Aminosäuren und hydrophoben anorganischen Pigmenten mit Antioxidantien neurosensorische Überempfindlichkeit und Juckreiz vermindern. Ferner war nicht vorauszusehen gewesen, daß sie zu hautverträglichen Produkten führen bzw. deren Verträglichkeit steigern und bei gesunder Haut nicht in die hauteigene Mikroflora eingreifen.

Die erfindungsgemäßen kosmetischen Zubereitungen können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feuchthaltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können die erfindungsgemäßen Zubereitungen außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1 bis 6 Gew.-% beträgt, bezogen auf das Gesamtgewicht der Zubereitung, um kosmetische Zubereitungen zur Verfügung zu stellen, die die Haut vor dem gesamten Bereich der ultravioletten Strahlung schützen, und in denen das Stinging der Aminosäuren verhindert oder drastisch vermindert ist.

Erfindungsgemaße Zubereitungen können auch vorteilhaft als Sonnenschutzmittel dienen.

Emulsionen gemäß der Erfindung z.B. in Form einer Sonnenschutzcreme oder einer Sonnenschutzmilch sind vorteilhaft und enthalten z.B. die genannten Fette, Öle, Wachse und anderen Fettkörper, sowie Wasser und einen Emulgator, wie er üblicherweise für einen solchen Typ der Formulierung verwendet wird.

Die UVB-Filter können öllöslich oder wasserlöslich sein. Vorteilhafte öllösliche UVB-Filter sind z.B.:

- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzalmalonsäuredi(2-ethylhexyl)ester;
- 2,4,6-Trianilino-(p-carbo-2'-ethyl-1'-hexyloxy)-1,3,5-triazin.

Vorteilhafte wasserlösliche UVB-Filter sind z.B.:

- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;

4

- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze.

Vorteilhaft können die erfindungsgemäßen Zubereitungen außerdem Substanzen enthalten, die UV-Strahlung im UVA-Bereich absorbieren. UVA-Filter, die erfindungsgemäß vorteilhaft verwendet werden können, sind beispielsweise Derivate des Dibenzoylmethans, insbesondere 1-(4'-tert.Butylphenyl)-3-(4'-methoxyphenyl)propan-1,3-dion und um 1-Phenyl-3-(4'-isopropylphenyl)propan-1,3-dion.

Die Gesamtmenge der UVA-Filtersubstanzen kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung, um kosmetische Zubereitungen zur Verfügung zu stellen, die die Haut vor dem gesamten Bereich der ultravioletten Strahlung schützen, und in denen das Stinging der Aminosäuren verhindert oder drastisch vermindert ist.

Vorteilhaft sind auch solche kosmetische und dermatologische Zubereitungen, die in der Form eines Sonnenschutzmittels, eines Pré-Soleil- oder Après-Soleil-Produktes vorliegen. Vorteilhaft enthalten diese zusätzlich mindestens einen UVA-Filter und/oder mindestens einen UVB-Filter.

Ferner sind auch solche kosmetische und dermatologische Zubereitungen besonders vorteilhaft, die in der Form eines Sonnenschutzmittels, eines Pré-Soleil- oder Après-Soleil-Produktes vorliegen und zusätzlich zu dem oder den UVA-Filtern und/oder dem oder den UVB-Filtern ein oder mehrere Antioxidantien enthalten.

Die Gesamtmenge der Antioxidantien kann vorteilhaft 0,1 Gew.-% bis 30 Gew.-%, vorzugsweise 0,5 bis 10 Gew.-%, insbesondere 1 bis 6 Gew.-% betragen, bezogen auf das Gesamtgewicht der Zubereitung.

Im den folgenden Beispielen werden vorteilhafte Verkörperungen der vorliegenden Erfindung aufgeführt.


## Beispiel 1

| Sonnencrème, O/W, Lichtschutzfaktor 20 | Gew.-% |
|---|---|
| Cyclomethicon | 3,00 |
| Glycerylstearat + PEG-30 Stearat | 2,00 |
| Wollwachsalkohol | 0,10 |
| Glycerylstearat | 3,00 |
| Isopropylpalmitat | 2,00 |
| Octyldodecanol | 1,00 |
| $C_{12-15}$Alkylbenzoat | 2,00 |
| Glycerin | 3,00 |
| Cetylalkohol | 3,00 |
| Myristylmyristat | 2,00 |
| Octylmethoxycinnamat | 4,50 |
| Butylmethoxydibenzoylmethan | 2,00 |
| Phenylbenzimidazolsulfonsäure | 3,00 |
| Tocopherylacetat | 0,50 |
| EDTA-Lösung (20 %-ig) | 0,50 |
| NaOH (45 %-ig) | 1,15 |

| | |
|---|---|
| Ethylalkohol | 4,00 |
| Konservierungsmittel | q.s. |
| Parfüm | q.s. |
| | |
| Hydrophobes TiO$_2$ | 2,00 |
| Glycin | 3,00 |
| | |
| Wasser, VES | ad 100,00 |

## Beispiel 2

| Sonnencrème, O/W, Lichtschutzfaktor 8 | Gew.-% |
|---|---|
| Cyclomethicon | 3,00 |
| Glycerylstearat + PEG-30 Stearat | 2,00 |
| Glycerylstearat | 3,00 |
| Isopropylpalmitat | 2,00 |
| Octyldodecanol | 3,00 |
| Glycerin | 3,00 |
| Cetylalkohol | 3,00 |
| Phenylbenzimidazolsulfonsäure | 1,00 |
| Octylmethoxycinnamat | 5,00 |
| Butylmethoxydibenzoylmethan | 0,50 |
| Tocopherylacetat | 0,50 |
| EDTA-Lösung (20 %-ig) | 0,50 |
| NaOH | q.s. |
| Ethylalkohol | 1,50 |
| Konservierungsmittel | q.s. |
| Parfüm | q.s. |
| | |
| Hydrophobes TiO$_2$ | 1,00 |
| Glycin | 0,50 |
| | |
| Wasser, VES | ad 100,00 |

6

## Beispiel 3

| Sonnenmilch, O/W | Gew.-% |
|---|---|
| Trilaureth-4 Phosphat | 0,75 |
| Triceteareth-4 Phosphat | 1,00 |
| Glycerylstearat + PEG-100 Stearat | 1,00 |
| Glycerylstearat + Ceteareth-20 | 0,80 |
| Glyceryllanolat | 0,50 |
| Isopropylpalmitat | 3,00 |
| Glycerin-Caprylat/Caproat | |
| ("Caprylic/Capric Triglyceride") | 5,00 |
| Cetylalkohol | 1,00 |
| Xanthangummi | 0,30 |
| Octylmethoxycinnamat | 6,00 |
| Butylmethoxydibenzoylmethan | 0,50 |
| Methylbenzylidencampher | 3,00 |
| Phenylbenzimidazolsulfonsäure | 4,00 |
| Butylhydroxytoluol | 0,06 |
| EDTA-Lösung (20 %-ig) | 0,50 |
| NaOH | q.s. |
| Konservierungsmittel | q.s. |
| Glycin | 1,00 |
| Hydrophobes $TiO_2$ | 3,00 |
| Wasser, VES | ad 100,00 |

## Beispiel 4

Sonnenmilch, O/W

| | Gew.-% |
|---|---|
| Cyclomethicon | 2,00 |
| Cetearylalkohol + PEG-40-hydriertes Rizinusöl + Natriumcetearylsulfat | 2,50 |

| | |
|---|---|
| Glyceryllanolat | 1,00 |
| Glycerin-Caprylat/Caproat | |
| ("Caprylic/Capric Triglyceride") | 2,00 |
| Octylstearat | 3,00 |
| Rizinusöl | 4,00 |
| Glycerin | 3,00 |
| Carbomer | 0,23 |
| Hydroxypropylmethylcellulose | 0,30 |
| PVP/Hexadecen Copolymer | 1,50 |
| Octylmethoxycinnamat | 4,00 |
| Butylmethoxydibenzoylmethan | 2,00 |
| Methylbenzylidencampher | 3,50 |
| Butylhydroxytoluol | 0,03 |
| EDTA-Lösung (20 %-ig) | 0,50 |
| NaOH | q.s. |
| Konservierungsmittel | q.s. |
| Parfüm | q.s. |
| | |
| Glycin | 2,00 |
| Hydrophobes $TiO_2$ | 1,50 |
| | |
| Wasser, VES | ad 100,00 |

## Beispiel 5

| Sonnenmilch, O/W | Gew.-% |
|---|---|
| Stearinsäure | 2,00 |
| Cetylpalmitat | 1,00 |
| Glycerin-Caprylat/Caproat | |
| ("Caprylic/Capric Triglyceride") | 2,00 |
| Sorbitanmonooleat | 1,00 |
| Sorbitanmonostearat | 1,50 |
| Paraffinöl, DAB 9 | 1,29 |
| Cetearylalkohol | 0,80 |

| | |
|---|---|
| Glycerin | 3,00 |
| 2-Hydroxy-4-methoxybenzophenon | 1,00 |
| Octylmethoxycinnamat | 5,00 |
| Methylbenzylidencampher | 1,00 |
| Butylhydroxytoluol | 0,06 |
| Simethicon | 0,20 |
| Polyethylenglycol-150 | 3,00 |
| Triethanolamin | 0,85 |
| Carbomer | 0,10 |
| Ethylalkohol | 3,00 |
| Konservierungsmittel | q.s. |
| Parfüm | q.s. |
| | |
| Glycin | 1,50 |
| Hydrophobes $TiO_2$ | 2,00 |
| | |
| Wasser, VES | ad 100,00 |

## Vergleichsbeispiel 1

| | |
|---|---|
| Sonnenmilch, O/W | Gew.-% |
| Cyclomethicon | 2,00 |
| Cetearylalkohol + PEG-40-hydriertes | |
| Rizinusöl + Natriumcetearylsulfat | 2,50 |
| Glyceryllanolat | 1,00 |
| Glycerin-Caprylat/Caproat | |
| ("Caprylic/Capric Triglyceride") | 2,00 |
| Octylstearat | 3,00 |
| Rizinusöl | 4,00 |
| Glycerin | 3,00 |
| Carbomer | 0,23 |
| Hydroxypropylmethylcellulose | 0,30 |
| PVP/Hexadecen Copolymer | 1,50 |
| Octylmethoxycinnamat | 4,00 |

9

| | |
|---|---|
| Butylmethoxydibenzoylmethan | 2,00 |
| Methylbenzylidencampher | 3,50 |
| Butylhydroxytoluol | 0,03 |
| Na$_3$HEDTA | 1,00 |
| NaOH | q.s. |
| Konservierungsmittel | q.s. |
| Parfüm | q.s. |
| Glycin | 2,00 |
| Wasser, VES | ad 100,00 |

## Versuchsbericht

Mit dem folgenden Versuchsbericht wird die Wirkung der erfindungsgemäßen Wirkstoffe demonstriert. Dazu wird die O/W-Emulsion des Beispiels 4, gegenüber dem Vergleichsbeispiel 1 (mit Glycin, aber ohne hydrophobes TiO$_2$) in einem "Stingingtest" als "Stinger" analog Frosch und Kligman verwendet (Peter J. Frosch und Albert M. Kligman, a.a.O).

Es wurden acht freiwillige Probanden ausgewählt, deren hohe Empfindlichkeit gegenüber der Aminosäure Glycin bekannt war. Alle Personen zeigten die erwartete Reaktion bei dem Vergleichsbeispiel. Fünf Personen teilten mit, daß die O/W-Emulsion gemäß Beispiel 4 geringere oder gar keine "Stinging-Effekte" hervorrief als bei der O/W-Emulsion gemäß Vergleichsbeispiel 1. Bei drei Personen waren keine Unterschiede zwischen der Anwendung von O/W-Emulsionen gemäß Beispiel 4 und Vergleichsbeispiel 1 festzustellen.

## Patentansprüche

1. Verwendung hydrophober anorganischer Pigmente zur Verhinderung des "Stingings" von Aminosäuren zur Herstellung von O/W-Emulsionen.

2. Verwendung nach Anspruch 1, dadurch gekennzeichnet, daß als Aminosäure oder als Aminosäuren Glycin und/oder Arginin gewählt wird oder werden, und/oder daß als hydrophobes anorganisches Pigment hydrophobes TiO$_2$ gewählt wird.

3. Verwendung nach Anspruch 1, dadurch gekennzeichnet, daß die O/W-Emulsionen einen Gehalt von 0,1 bis 10 Gew.-% an Aminosäuren und/oder einen Gehalt von 0,1 bis 10 Gew.-% an hydrophoben anorganischen Pigmenten aufweisen, jeweils bezogen auf das Gesamtgewicht der Zusammensetzung.

4. Verwendung nach Anspruch1, dadurch gekennzeichnet, daß die O/W-Emulsionen einen Gehalt von jeweils 0,01 bis 30 Gew.-% an einer oder mehreren UV-A-Filtersubstanzen und/oder einer oder mehreren UV-B-Filtersubstanzen und/oder einem oder mehreren Antioxidantienaufweisen, jeweils bezogen auf das Gesamtgewicht der Zusammensetzung.

## Claims

1. Use of hydrophobic inorganic pigments for the prevention of "stinging" of amino acids for the preparation of O/W emulsions.

2. Use according to Claim 1, characterized in that the amino acid or the amino acids selected is/are glycine and/or arginine, and/or in that the hydrophobic inorganic pigment selected is hydrophobic TiO$_2$.

3. Use according to Claim 1, characterized in that the O/W emulsions have a content of 0.1 to 10% by weight of amino acids and/or a content of 0.1 to 10% by weight of hydrophobic inorganic pigments, in each case based on the total weight of the composition.

4. Use according to Claim 1, characterized in that the O/W emulsions have a content of 0.01 to 30% by weight in each case of one or more UV-A filter substances and/or one or more UV-B filter substances and/or one or more antioxidants, in each case based on the total weight of the composition.

**Revendications**

1. Utilisation de pigments inorganiques hydrophobes pour empêcher le "stinging" d'acides aminés dans des émulsions H/E.

2. Utilisation selon la revendication 1, caractérisée en ce que l'on choisit, en tant qu'acide aminé ou acides aminés, la glycine et/ou l'arginine, et/ou en ce que l'on choisit, en tant que pigment inorganique hydrophobe, du TiO$_2$.

3. Utilisation selon la revendication 1, caractérisée en ce que les émulsions H/E présentent une teneur de 0,1 à 10 % en poids en acides aminés et/ou une teneur de 0,1 à 10 % en poids de pigments hydrophobes, à chaque fois par rapport au poids total de la composition..

4. Utilisation selon la revendication 1, caractérisée en ce que les émulsions H/E présentent une teneur de 0,01 à 30% en poids à chaque fois, en une ou plusieurs substances filtrantes UV-A et/ou en une ou plusieurs substances filtrantes UV-B et/ou en un ou plusieurs antioxydants, à chaque fois par rapport au poids total de la composition.